# EUROPEAN PATENT APPLICATION

(11) **EP 2 196 190 A1**
(43) Date of publication of application: **16.06.2010**
(21) Application number: 08021350.7
(22) Date of filing: 09.12.2008
(51) Int. Cl.: A61K 8/49, A61Q 19/04, A61Q 19/08

(54) **Skin whitening**

(71) Applicant: Cognis IP Management GmbH, 49589 Düsseldorf (DE)
(72) Inventor: Moussou, Philippe, 54510, Tomlaine (FR); Sabadotto, Melanie, 54130, St. Max (FR); Rathjens, Andreas, 54510, Tomblaine (FR)

(57) **Abstract**

Use of a substance of formula (I) 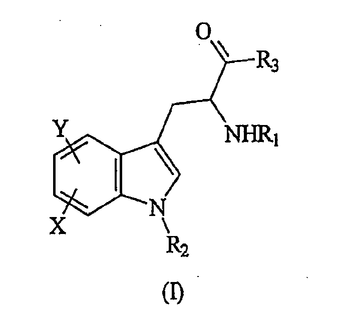 in the D-form, L-form or racemic form (D,L),
wherein
- R₁ is selected from the group consisting of hydrogen group (-H), methyl group (-CH₃), ethyl group (-CH₂CH₃), C3 alkyl group, C4 alkyl group, acetyl group (-C(O)CH₃), propanoyl group (-C(O)CH₂CH₃), n-butanoyl group (-C(O)CH₂CH₂CH₃), and 2-methyl-propanoyl group (-C(O)CH(CH₃)₂),
- R₂ is selected from the group consisting of hydrogen group (-H), ethyl group (-CH₂CH₃), C3 alkyl group, C4 alkyl group, acetyl group (-C(O)CH₃), propanoyl group (-C(O)CH₂H₃), n-butanoyl group (-C(O)CH₂CH₂CH₃), and 2-methyl-propanoyl group (-C(O)CH(CH₃)₂),
- R₃ is selected from the group consisting of hydroxyl group (-OH), amino group (-NH₂, methoxy group (OCH₃), ethoxy group (-OCH₂CH₃), C3 alkoxy group, C4 alkoxy group, and benzyloxy group (-OCH₂C₆H₅),
- X and Y are independently selected from the group consisting of hydrogen group (-H), methyl group (-CH₃), ethyl group (-CH₂CH₃), C3 group alkyl, C4 alkyl group, halogen group, methoxy group (-OCH₃), ethoxy group (-OCH₂H₃) C3 alkoxy group, C4 alkoxy group, and benzyloxy group (-OCH₂C₆H₅,

with the proviso that at least one of the residues X and Y is not a hydrogen group (-H),
(a) for the manufacture of cosmetic compositions and/or topical compositions

## Description

The present invention relates to tryptophan derivatives for use in cosmetics, especially for skin whitening and/or against the signs of aging skin. The present invention also relates to the use of such substances for the treatment of disorders related to the pigmentation of the skin.

There are a number of cosmetic compositions available to consumers to improve the health and aspect of the skin. In particular, these cosmetics are used to improve skin tone, lighten or reduce skin pigmentation, prevent or treat pigmentation disorders such as hyperpigmentation and aged spots. Hyperpigmentation diseases include, for example, chloasma (a hypersecretion of melanin induced by hormonam factors and amplified by the effects of sun exposure), lentigines, solar and senile lentigo, Dubreuilh melanosis, melasma, or any form of hypermelanosis or melanocyte dysfunction.

Skin hyperpigmentation such as spots, freckles, and liver spots are caused by sunburn and the like. It increases or becomes harder to fade with aging, a problem which annoys in particular the middle aged and older. Such pigmentation, for which the mechanism is yet to be established, is believed to be caused by inflammation of the skin as induced by sunlight (in particular, ultraviolet rays) or the like.

The melanin pigment is produced in the melanin-producing granule, which is called melanosome, in the melanocytes present in the epidermis and is delivered to adjacent epidermal cells.

The above problem has prompted the development of a substance capable of modifying such pigmentation, restoring a normal skin color, whitening and/or lightening of the skin, resulting in many commercial products.

Several compounds are known in the cosmetic industry to be used to decrease skin pigmentation, such as hydroquinone, arbutin, kojic acid, azelaic acid, ascorbic acid or its derivatives such as magnesium ascorbyl phosphate, some plant extracts such as Morus alba extracts or Glycyrrhiza glabra. However, these products may be cytotoxic, poor efficacy, difficult to use in formulation due to instability, or dark color impact. For example kojic acid is unstable in cosmetic formulations leading to dark-brown products. Further in several countries the use of hydroquinone is forbidden in cosmetics.

It is known from FR 2 886 843 that the use of a Griffonia simplicifolia extract containing 5-hydroxytryptophan decreases the melanin production by melanocytes in cell culture, and that Griffonia simplicifolia extract can be used to decrease skin pigmentation.

Similar inhibitory effect on melanin formation in B16 melanocytes cells is described by KT Kim et al (Kim, KT, et al "5-hydroxytryptophan as a novel inhibitor of melanin synthesis"; IFSCC Congress 2006. PC-026).

On the other hand S. Vogliardi et al reports that tyrosinase, the key catalytic enzyme in the melanin synthesis pathway, is able to activate melanogenesis starting from 5-hydroxytrypthophan (Vogliardi, S, et al "an investigation of the role of 5-hydroxytrypthophan in the biosynthesis of melanins"; J Mass Spectrometry, 2002, 37, 1292 - 6).

Thus there is to date an uncertainty whether the tryptophan derivatives can be effectively applied in the reduction of pigmentation.

Therefore, there is a need for a safe, stable, and easy way to formulate cosmetically effective depigmenting or lightening active ingredients that seek to improve the skin appearance and to remedy these pigmentation conditions. In addition, some dark-skinned individuals prefer a light skin color, as it is regarded as a particular beauty feature.

The finding of the present invention is to use specific tryptophan derivatives substituted at the indole residue for skin whitening and/or reduction of pigmentation.

Thus, the present invention is directed to the use of a substance of formula (I) 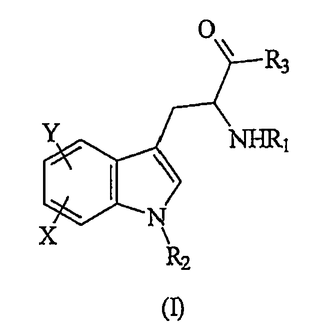 in the D-form, L-form or racemic form (D,L),
wherein
- R₁ is selected from the group consisting of hydrogen group (-H), methyl group (-CH₃), ethyl group (-CH₂CH₃), C3 alkyl group, C4 alkyl group, acetyl group (-C(O)CH₃), propanoyl group (-C(O)CH₂CH₃), n-butanoyl group (-C(O)CH₂CH₂CH₃), and 2-methyl-propanoyl group (-C(O)CH(CH₃)₂),
- R₂ is selected from the group consisting of hydrogen group (-H), ethyl group (-CH₂CH₃), C3 alkyl group, C4 alkyl group, acetyl group (-C(O)CH₃), propanoyl group (-C(O)CH₂CH₃), n-butanoyl group (-C(O)CH₂CH₂CH₃), and 2-methyl-propanoyl group (-C(O)CH(CH₃)₂),
- R₃ is selected from the group consisting of hydroxyl group (-OH), amino group (-NH₂), methoxy group (OCH₃), ethoxy group (-OCH₂CH₃), C3 alkoxy group, C4 alkoxy group, and benzyloxy group (-OCH₂C₆H₅),
- X and Y are independently selected from the group consisting of hydrogen group (-H), methyl group (-CH₃), ethyl group (-CH₂CH₃), C3 group alkyl, C4 alkyl group, halogen group, methoxy group (-OCH₃), ethoxy group (-OCH₂CH₃), C3 alkoxy group, C4 alkoxy group, and benzyloxy group (-OCH₂C₆H₅),
with the proviso that at least one of the residues X and Y is not a hydrogen group (-H),
(a) for the manufacture of cosmetic compositions and/or topical compositions and/or
(b) in cosmetic compositions and/or topical compositions
   for
   (i) the lightening of the skin and/or
   (ii) the whitening of the skin and/or
   (iii) the reduction of pigmentation of the skin and/or
   (iv) the reduction of hyperpigmentation of the skin and/or
   (v) the inhibition of melanogenesis in the skin and/or
   (vi) the prevention and/or retardation of signs of ageing and/or improving the skin appearance of an aged skin.

It was surprisingly found out that the substances of formula (I) reduces melanin synthesis in melanocytes. Further, the substances of formula (I) in contrast to other known tryptophan derivatives, in particular in comparison to 5-hydroxytryptophan, are more stable in aqueous solutions and thus can effectively decrease the synthesis of melanin in melanocytes without cell toxicity also after storage time. After few days, aqueous solutions of 5-hydroxytryptophan leads to the formation a dark-brown color, which is particularly unsuitable for cosmetic composition to improve the skin lightness, or to decrease skin dark spots. Surprisingly, with the substances of formula (I) such coloration does not occur. As the substances of formula (I) inhibit the generation of melanin, the cosmetic compositions comprising said substances can improve skin pigmentation and can be used in particular for whitening and/or lightening cosmetics and/or can prevent pigmentation and/or can inhibit melanogenesis of the skin.

Thus, the present invention relates, in particular, to the use of the substance of formula (I) as a melanogenesis inhibitor and/or whitening agent.

The terms "lightening of the skin" and "whitening of the skin" indicate in particular the effect achieved by the substance of formula (I). Accordingly, "lightening" or "whitening" preferably stands for the change in the color of the skin to a color which is lighter compared to the state before treatment with the substance of formula (I) according to the invention. The use of the substance according to formula (I) thus preferably encompasses the removal or reduction of pigmentation, e.g. of spots and/or of freckles, of hyperpigmentation which might be caused by exaggerated sun exposure but also the lightening of the cited pigments, e.g. changing the skin tone to a lighter one or lightening of freckles, etc.

The term "reduction" as used in connection with pigmentation or hyperpigmentation indicates that the amount of pigments, such as spots, freckles, and/or liver spots, preferably melanin in the skin, is reduced compared to the skin before having been treated with a substance according to formula (I) according to the present invention.

Melanogenesis is the production and delivery of melanin by melanocytes in human skin. It is (mainly) stimulated by UV radiation, and it leads to a delayed development of a tan. This melanogenesis-based tan takes more time to develop but is long lasting than immediate sun-tanning. Accordingly, inhibition of melanogenesis of the skin is preferably understood as the prevention of melanin synthesis or at least a suppression of melanin synthesis. Suppression in this context means the decrease of melanin synthesis by melanocytes of at least 10%, more preferably at least 20%, yet more preferably at least 50%, still yet more preferably at least 80%, like about 100%, in the skin compared to a skin not treated with the substance according to formula (I) according to this invention.

Pigmentation disorders and or hyperpigmentation diseases according to this invention include disorders or diseases which exhibit an abnormal melanogenesis. It includes in particular chloasma (a hypersecretion of melanin induced by hormonam factors and amplified by the effects of sun exposure), lentigines, solar and senile lentigo, Dubreuilh melanosis, melasma, or any form of hypermelanosis or melanocyte dysfunction.

Prevention and/or retardation of signs of aging mean, in particular, that the use of the substance according to formula (I) inhibits or delays (postpones) skin aging, preferably skin aging in the form of formation of age spots, brown spots, and/or liver spots, wrinkles, uneven skin tone, and/or loss of skin elasticity compared to a non-treated, aged skin.

"Improving the skin appearance of the aged skin" is preferably understood as the improved visual appearance of skin, in particular, the reduction of age spots, brown spots, liver spots, wrinkles, uneven skin tone, and/or loss of skin elasticity, compared to a non-treated, aged skin.

Preferred substances of formula (I) are those wherein
- R₁ is selected from the group consisting of hydrogen group (-H), methyl group (-CH₃), ethyl group (-CH₂CH₃), n-propyl group (-CH₂CH₂CH₃), iso-propyl group (-C(CH₃)₂), n-butyl group (-CH₂CH₂CH₂CH₃), 2-methyl-propanyl group (-CH₂CH(CH₃)CH₃), acetyl group (-C(O)CH₃), propanoyl group (-C(O)CH₂CH₃), n-butanoyl group (-C(O)CH₂CH₂CH₃), and 2-methyl-propanoyl group (-C(O)CH(CH₃)₂),
- R₂ is selected from the group consisting of hydrogen group (-H), ethyl group (-CH₂CH₃), n-propyl group (-CH₂CH₂CH₃), iso-propyl group (-C(CH₃)₂), n-butyl group (-CH₂CH₂CH₂CH₃), 2-methyl-propanyl group (-CH₂CH(CH₃)CH₃), acetyl group (-C(O)CH₃), propanoyl group (-C(O)CH₂CH₃), n-butanoyl group (-C(O)CH₂CH₂CH₃), and 2-methyl-propanoyl group (-C(O)CH(CH₃)₂),
- R₃ is selected from the group consisting of hydroxyl group (-OH), amino group (-NH₂), methoxy group (OCH₃), ethoxy group (-OCH₂CH₃), C3 alkoxy group, C4 alkoxy group, and benzyloxy group (-OCH₂C₆H₅),
- X and Y are independently selected from the group consisting of hydrogen group (-H), methyl group (-CH₃), ethyl group (-CH₂CH₃), n-propyl group (-CH₂CH₂CH₃), iso-propyl group (-C(CH₃)₂), n-butyl group (-CH₂CH₂CH₂CH₃), 2-methyl-propanyl group (-CH₂CH(CH₃)CH₃), fluorine group (-F), bromine group (-Br), chlorine (-Cl), methoxy group (-OCH₃), ethoxy group (-OCH₂CH₃), C3 alkoxy group, C4 alkoxy group, and benzyloxy group (-OCH₂C₆H₅), with the proviso that at least one of the residues X and Y is not a hydrogen group (-H),

More preferred substances of formula (I) are those wherein
- R₁ is selected from the group consisting of hydrogen group (-H), methyl group (-CH₃), ethyl group (-CH₂CH₃), n-propyl group (-CH₂CH₂CH₃), and isopropyl group (-C₂(CH₃)₂),
- R₂ is selected from the group consisting of hydrogen group (-H), methyl group (-CH₃), ethyl group (-CH₂CH₃), n-propyl group (-CH₂CH₂CH₃), and isopropyl group (-C₂(CH₃)₂),
- R₃ is selected from the group consisting of hydroxyl group (-OH), amino group (-NH₂), methoxy group (OCH₃), ethoxy group (-OCH₂CH₃), and benzyloxy group (-OCH₂C₆H₅),
- X and Y are independently selected from the group consisting of hydrogen group (-H), methyl group (-CH₃), fluorine group (-F), bromine group (-Br), methoxy group (-OCH₃), and ethoxy group (-OCH₂CH₃), with the proviso that at least one of the residues X and Y is not a hydrogen group (-H).

Yet more preferred substances of formula (I) are those wherein
- R₁ is either a hydrogen group (-H) or a methyl group (-CH₃),
- R₂ is either hydrogen group (-H) or methyl group (-CH₃),
- R₃ is either hydroxyl group (-OH) or amino group (-NH₂),
- Y is a hydrogen group (-H), and
- X is selected from the group consisting of methyl group (-CH₃), fluorine group (-F), and methoxy group (-OCH₃).

Still yet more preferred substances of formula (I) are those wherein
- R₁ and R₂ are hydrogen groups (-H),
- R₃ is a hydroxyl group (-OH),
- Y is a hydrogen group (-H), and
- X is either a methyl group (-CH₃) or fluorine group (-F).

Especially preferred substances are those in which formula (I) is defined as follows:
- R₁ and R₂ are hydrogen groups (-H),
- R₃ is a hydroxyl group (-OH),
- Y is a hydrogen group (-H), and
- X is either a methyl group (-CH₃) or fluorine group (-F) and X is in the 4, 5 or 6 position of the tryptophan ring.

Accordingly especially suitable is 6-fluoro-D,L-trypthophan and/or 4-methyl-D,L-trypthophan.

The substance according to formula (I) can be used alone or together with other known agents used in the technical field of the present invention. Accordingly the substances according to formula (I) can be used together with at least one member selected from the group consisting of kojic acid, hydroquinone, alpha- and beta-arbutin, other hydroquinone glycosides, deoxyarbutin, ferulic acid, diacetyl-boldine, azelaic acid, octadecenedioic acid, linoleic acid, conjugated linoleic acid, alpha-lipoic acid, glutathione and derivatives, undecylenoyl-phenylalanine, vitamin C and derivatives as magnesium L-ascorbyl-phosphate, niacinamide, 4-n-butyl-resorcinol, alpha- and beta-hydroxy acids, ellagic acid, resveratrol, Morus alba extracts, glabridin and liquorice extracts, imperatorin and isoimperatorin and Angelica dahurica extracts, centaureidin and Yarrow extracts, Bellis perennis extracts, Phyllanthus emblica extracts, water cress extracts, Veratum nigrum extracts, Sophora flavescens extracts, ascomycete-derived melanin-degrading enzyme, acetoxysinapinic acid and 2-(4-hydroxyphenoxy)propionic acid.

The present invention is further directed to the substance according to formula (I) as defined in the present invention for the treatment of a disease connected to a disorder in the pigmentation of the skin. More preferably, the substance according to formula (I) as defined in the present invention is directed to a disease exhibiting an abnormal melanin synthesis and/or secretion, like chloasma (a hypersecretion of melanin induced by hormonam factors and amplified by the effects of sun exposure), lentigines, solar and senile lentigo, Dubreuilh melanosis, melasma, or any form of hypermelanosis or melanocyte dysfunction.

In a further aspect, the present invention is directed to a cosmetic composition and/or a topical composition comprising the substance according to formula (I) as defined in the instant invention.

The cosmetic composition and/or the topical composition may comprise the substance according to formula (I) of the present invention in an amount of 0.0001 to 10 wt.-%, more preferably in an amount of 0.002 to 2 %, yet more preferably in an amount of 0.003 to 1 % based on the total amount of the composition.

The term "cosmetics" includes medicated cosmetics such as dermatological preparations, ointments, solutions, creams, emulsions, toners, lotions, gels, essences, foundations, pack masks, lipsticks, sticks, bath preparations, and the like.

The cosmetic form can encompass a broad range of formulation types such as solution solubilized formula, powders, powder dispersions, oily solutions, gels, ointments, aerosols, water-in-oil, water-in-oil-in-solid types, and the like.

The cosmetic composition according to this invention can be especially in the form of hair shampoos, hair lotions, foam baths, shower bath creams, gels, lotions, alcoholic and aqueous/alcoholic solutions, emulsions, wax/fat masses, stick preparations, powders, or ointments. These compositions can also comprise further auxiliaries and additives, mild surfactants, oil bodies, emulsifiers, pearlescent waxes, consistency regulators, thickeners, superfatting agents, stabilizers, polymers, silicone compounds, fats, waxes, lecithins, phospholipids, UV photoprotective factors, biogenic active ingredients, antioxidants, deodorants, antiperspirants, antidandruff agents, film formers, swelling agents, insect repellents, self-tanning agents, hydrotropes, solubilizers, preservatives, perfume oils, dyes and the like.

The inventive cosmetic composition may further comprise at least one surfactant.

Surface-active substances which may be present are anionic, nonionic, cationic and/or amphoteric or zwitterionic surfactants, the content of which in the compositions is usually about 1 to 70% by weight, preferably 5 to 50% by weight and in particular 10 to 30% by weight. Typical examples of anionic surfactants are soaps, alkylbenzenesulphonates, alkanesulphonates, olefinsulphonates, alkyl ether sulphonates, glycerol ether sulphonates, α-methyl ester sulphonates, sulpho fatty acids, alkyl sulphates, alkyl ether sulphates, glycerol ether sulphates, fatty acid ether sulphates, hydroxy mixed ether sulphates, monoglyceride (ether) sulphates, fatty acid amide (ether) sulphates, mono- and dialkyl sulphosuccinates, mono- and dialkyl sulphosuccinamates, sulphotriglycerides, amide soaps, ether carboxylic acids and salts thereof, fatty acid isethionates, fatty acid sarcosinates, fatty acid taurides, N-acylaminoacids, such as, for example, acyl lactylates, acyl tartrates, acyl glutamates and acyl aspartates, alkyl oligoglucoside sulphates, protein fatty acid condensates (in particular wheat-based vegetable products) and alkyl (ether) phosphates. If the anionic surfactants comprise polyglycol ether chains, these can have a conventional homologue distribution, but preferably have a narrowed homologue distribution. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partially oxidized alk(en)yl oligoglycosides and glucoronic acid derivatives, fatty acid N-alkylglucamides, protein hydrolysates (in particular wheat-based vegetable products), polyol fatty acid esters, sugar esters, sorbitan esters, polysorbates and amine oxides. If the nonionic surfactants contain polyglycol ether chains, these can have a conventional homologue distribution, but preferably have a narrowed homologue distribution. Typical examples of cationic surfactants are quaternary ammonium compounds, such as, for example, dimethyldistearylammonium chloride, and ester quats, in particular quaternized fatty acid trialkanolamine ester salts. Typical examples of amphoteric and zwitterionic surfactants are alkylbetaines, alkylamidobetaines, aminopropionates, aminoglycinates, imidazoliniumbetaines and sulphobetaines. The specified surfactants are exclusively known compounds. Typical examples of particularly suitable mild, i.e. particularly skin-compatible, surfactants are fatty alcohol polyglycol ether sulphates, monoglyceride sulphates, mono- and/or dialkyl sulphosuccinates, fatty acid isethionates, fatty acid sarcosinates, fatty acid taurides, fatty acid glutamates, α-olefinsulphonates, ether carboxylic acids, alkyl oligoglucosides, fatty acid glucamides, alkylamidobetaines, amphoacetals and/or protein fatty acid condensates, the latter preferably being based on wheat proteins.

Additionally or alternatively the inventive cosmetic composition may further comprise at least one oil body.

Suitable oil bodies are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols and/or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkyl hydroxycarboxylic acids with linear or branched C₆-C₂₂-fatty alcohols in particular dioctyl malates, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, dicaprylyl carbonate (Cetiol^{®} CC), Guerbet carbonates based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or unsymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicon methicone types, inter alia) and/or aliphatic or naphthenic hydrocarbons, such as, for example, squalane, squalene or dialkyleyclohexanes.

Additionally or alternatively the inventive cosmetic composition may further comprise at least one emulsifier.

Suitable emulsifiers are, for example, nonionogenic surfactants from at least one of the following groups:
- addition products of from 2 to 30 mol of ethylene oxide and/or 0 to 5 mol of propylene oxide to linear fatty alcohols having 8 to 22 carbon atoms, to fatty acids having 12 to 22 carbon atoms, to alkylphenols having 8 to 15 carbon atoms in the alkyl group, and alkylamines having 8 to 22 carbon atoms in the alkyl radical;
- alkyl and/or alkenyl oligoglycosides having 8 to 22 carbon atoms in the alk(en)yl radical and the ethoxylated analogues thereof;
- addition products of from 1 to 15 mol of ethylene oxide to castor oil and/or hydrogenated castor oil;
- addition products of from 15 to 60 mol of ethylene oxide to castor oil and/or hydrogenated castor oil;
- partial esters of glycerol and/or sorbitan with unsaturated, linear or saturated, branched fatty acids having 12 to 22 carbon atoms and/or hydroxycarboxylic acids having 3 to 18 carbon atoms, and the adducts thereof with 1 to 30 mol of ethylene oxide;
- partial esters of polyglycerol (average degree of self-condensation 2 to 8), polyethylene glycol (molecular weight 400 to 5 000), trimethylolpropane, pentaerythritol, sugar alcohols (e.g. sorbitol), alkyl glucosides (e.g. methyl glucoside, butyl glucoside, lauryl glucoside), and polyglucosides (e.g. cellulose) with saturated and/or unsaturated, linear or branched fatty acids having 12 to 22 carbon atoms and/or hydroxycarboxylic acids having 3 to 18 carbon atoms, and the adducts thereof with 1 to 30 mol of ethylene oxide;
- mixed esters of pentaerythritol, fatty acids, citric acid and fatty alcohol and/or mixed esters of fatty acids having 6 to 22 carbon atoms, methylglucose and polyols, preferably glycerol or polyglycerol,
- mono-, di- and trialkyl phosphates, and mono-, di- and/or tri-PEG alkyl phosphates and salts thereof;
- wool wax alcohols;
- polysiloxane-polyalkyl-polyether copolymers and corresponding derivatives;
- block copolymers, e.g. polyethylene glycol-30 dipolyhydroxystearates;
- polymer emulsifiers, e.g. Pemulen grades (TR-1, TR-2) from Goodrich;
- polyalkylene glycols, and
- glycerol carbonate.

### • Ethylene oxide addition products

The addition products of ethylene oxide and/or of propylene oxide to fatty alcohols, fatty acids, alkylphenols or to castor oil are known, commercially available products. These are homologue mixtures whose average degree of alkoxylation corresponds to the ratio of the amounts of substance of ethylene oxide and/or propylene oxide and substrate with which the addition reaction is carried out. C_{12/18}-fatty acid mono- and diesters of addition products of ethylene oxide to glycerol are known as refatting agents for cosmetic preparations.

### • Alkyl and/or alkenyl oligoglycosides

Alkyl and/or alkenyl oligoglycosides, their preparation and their use are known from the prior art. They are prepared, in particular, by reacting glucose or oligosaccharides with primary alcohols having 8 to 18 carbon atoms. With regard to the glycoside radical, both monoglycosides, in which a cyclic sugar radical is glycosidically bonded to the fatty alcohol, and also oligomeric glycosides having a degree of oligomerization of up to, preferably, about 8, are suitable. The degree of oligomerization here is a statistical average value which is based on a homologue distribution customary for such technical-grade products.

### • Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride, and the technical-grade mixtures thereof which may also comprise small amounts of triglyceride as a minor product of the preparation process. Likewise suitable are addition products of 1 to 30 mol, preferably 5 to 10 mol, of ethylene oxide to said partial glycerides.

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate, and technical-grade mixtures thereof. Likewise suitable are addition products of from 1 to 30 mol, preferably 5 to 10 mol, of ethylene oxide to said sorbitan esters.

### • Polyglycerol esters

Typical examples of suitable polyglycerol esters are polyglyceryl-2 dipolyhydroxystearate (Dehymuls^{®} PGPH), polyglycerol-3 diisostearate (Lameform^{®} TGI), polyglyceryl-4 isostearate (Isolan^{®} GI 34), polyglyceryl-3 oleate, diisostearoyl polyglyceryl-3 diisostearate (Isolan^{®} PDI), polyglyceryl-3 methylglucose distearate (Tego Care^{®} 450), polyglyceryl-3 beeswax (Cera Bellina^{®}), polyglyceryl-4 caprate (Polyglycerol Caprate T2010/90), polyglyceryl-3 cetyl ether (Chimexane^{®} NL), polyglyceryl-3 distearate (Cremophor^{®} GS 32) and polyglyceryl polyricinoleate (Admul^{®} WOL 1403), polyglyceryl dimerate isostearate, and mixtures thereof. Examples of further suitable polyol esters are the mono-, di- and triesters, optionally reacted with 1 to 30 mol of ethylene oxide, of trimethylolpropane or pentaerythritol with lauric acid, coconut fatty acid, tallow fatty acid, palmitic acid, stearic acid, oleic acid, behenic acid and the like.

### • Anionic emulsifiers

Typical anionic emulsifiers are aliphatic fatty acids having 12 to 22 carbon atoms, such as, for example, palmitic acid, stearic acid or behenic acid, and dicarboxylic acids having 12 to 22 carbon atoms, such as, for example, azelaic acid or sebacic acid.

### • Amphoteric and cationic emulsifiers

Furthermore, zwitterionic surfactants can be used as emulsifiers. The term "zwitterionic surfactants" refers to those surface-active compounds which carry at least one quaternary ammonium group and at least one carboxylate and one sulphonate group in the molecule. Particularly suitable zwitterionic surfactants are the so-called betaines, such as N-alkyl-N,N-dimethylammonium glycinates, for example cocoalkyldimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines having in each case 8 to 18 carbon atoms in the alkyl or acyl group, and cocoacylaminoethylhydroxyethylcarboxymethyl glycinate. Particular preference is given to the fatty acid amide derivative known under the CTFA name *Cocamidopropyl Betaine*. Likewise suitable emulsifiers are ampholytic surfactants. The term "ampholytic surfactants" means those surface-active compounds which, apart from a C_{8/18}-alkyl or -acyl group, contain at least one free amino group and at least one -COOH or -SO₃H group in the molecule and are capable of forming internal salts. Examples of suitable ampholytic surfactants are N-alkylglycines, N-alkylaminopropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids having in each case about 8 to 18 carbon atoms in the alkyl group. Particularly preferred ampholytic surfactants are N-cocoalkylaminopropionate, cocoacylaminoethylaminopropionate and C_{12/18}-acylsarcosine. Finally, cationic surfactants are also suitable as emulsifiers, those of the ester quat type, preferably methyl-quaternized difatty acid triethanolamine ester salts, being particularly preferred.

In one embodiment of the invention the cosmetic composition further comprises at least one fat or wax.

Typical examples of fats are glycerides, i.e. solid or liquid vegetable or animal products which consist essentially of mixed glycerol esters of higher fatty acids, suitable waxes are inter alia natural waxes, such as, for example, candelilla wax, carnauba wax, Japan wax, esparto grass wax, cork wax, guaruma wax, rice germ oil wax, sugarcane wax, ouricury wax, montan wax, beeswax, shellac wax, spermaceti, lanolin (wool wax), uropygial grease, ceresin, ozokerite (earth wax), petrolatum, paraffin waxes, microcrystalline waxes; chemically modified waxes (hard waxes), such as, for example, montan ester waxes, sasol waxes, hydrogenated jojoba waxes, and synthetic waxes, such as, for example, polyalkylene waxes and polyethylene glycol waxes. In addition to the fats, suitable additives are also fat-like substances, such as lecithins and phospholipids. The term lecithins is understood by the person skilled in the art as meaning those glycerophospholipids which are founded from fatty acids, glycerol, phosphoric acid and choline by esterification. Lecithins are thus also often as phosphatidylcholines (PC) in the specialist world. Examples of natural lecithins which may be mentioned are the cephalins, which are also referred to as phosphatidic acids and constitute derivatives of 1,2-diacyl-sn-glycerol-3-phosphoric acids. By contrast, phospholipids are usually understood as meaning mono- and preferably diesters of phosphoric acid with glycerol (glycerol phosphates), which are generally classed as fats. In addition, sphingosines or sphingolipids are also suitable.

In one embodiment of the invention the cosmetic composition further comprises at least one pearlescent wax.

Examples of suitable pearlescent waxes are: alkylene glycol esters, specifically ethylene glycol distearate; fatty acid alkanolamides, specifically coconut fatty acid diethanolamide; partial glycerides, specifically stearic acid monoglyceride; esters of polybasic, optionally hydroxy-substituted carboxylic acids with fatty alcohols having 6 to 22 carbon atoms, specifically long-chain esters of tartaric acid; fatty substances, such as, for example, fatty alcohols, fatty ketones, fatty aldehydes, fatty ethers and fatty carbonates, which have a total of at least 24 carbon atoms, specifically laurone and distearyl ether; fatty acids, such as stearic acid, hydroxystearic acid or behenic acid, ring-opening products of olefin epoxides having 12 to 22 carbon atoms with fatty alcohols having 12 to 22 carbon atoms and/or polyols having 2 to 15 carbon atoms and 2 to 10 hydroxyl groups, and mixtures thereof.

In one embodiment of the invention the cosmetic composition further comprises at least one consistency regulator and/or thickener.

Suitable consistency regulators are primarily fatty alcohols or hydroxy fatty alcohols having 12 to 22, and preferably 16 to 18, carbon atoms, and also partial glycerides, fatty acids or hydroxy fatty acids. Preference is given to a combination of these substances with alkyl oligoglucosides and/or fatty acid N-methylglucamides of identical chain length and/or polyglycerol poly-12-hydroxystearates. Suitable thickeners are, for example, Aerosil grades (hydrophilic silicas), polysaccharides, in particular xanthan gum, guar guar, agar agar, alginates and tyloses, carboxymethylcellulose, hydroxyethylcellulose and hydroxypropylcellulose, and also relatively high molecular weight polyethylene glycol mono- and diesters of fatty acids, polyacrylates (e.g. Carbopols^{®} and Pemulen grades from Goodrich; Synthalens^{®} from Sigma; Keltrol grades from Kelco; Sepigel grades from Seppic; Salcare grades from Allied Colloids), polyacrylamides, polymers, polyvinyl alcohol and polyvinylpyrrolidone. Bentonites, such as, for example, Bentone^{®} Gel VS 5PC (Rheox), which is a mixture of cyclopentasiloxane, disteardimonium hectorite and propylene carbonate, have also proven to be particularly effective. Also suitable are surfactants, such as, for example, ethoxylated fatty acid glycerides, esters of fatty acids with polyols such as, for example, pentaerythritol or trimethylolpropane, fatty alcohol ethoxylates having a narrowed homologue distribution or alkyl oligoglucosides, and electrolytes such as sodium chloride and ammonium chloride.

In one embodiment of the invention the cosmetic composition further comprises at least one superfatting agent.

Superfatting agents which can be used are substances such as, for example, lanolin and lecithin, and polyethoxylated or acylated lanolin and lecithin derivatives, polyol fatty acid esters, monoglycerides and fatty acid alkanolamides, the latter also serving as foam stabilizers.

In one embodiment of the invention the cosmetic composition further comprises at least one stabilizer.

Stabilizers which can be used are metal salts of fatty acids, such as, for example, magnesium, aluminium and/or zinc stearate or ricinoleate.

In one embodiment of the invention the cosmetic composition further comprises at least one polymer.

Suitable cationic polymers are, for example, cationic cellulose derivatives, such as, for example, a quaternized hydroxyethylcellulose obtainable under the name Polymer JR 400^{®} from Amerchol, cationic starch, copolymers of diallylammonium salts and acrylamides, quaternized vinylpyrrolidone-vinylimidazole polymers, such as, for example, Luviquat^{®} (BASF), condensation products of polyglycols and amines, quaternized collagen polypeptides, such as, for example, lauryldimonium hydroxypropyl hydrolyzed collagen (Lamequat^{®}L/Grünau), quaternized wheat polypeptides, polyethyleneimine, cationic silicone polymers, such as, for example, amodimethicones, copolymers of adipic acid and dimethylaminohydroxypropyldiethylenetriamine (Cartaretins^{®}/Sandoz), copolymers of acrylic acid with dimethyldiallylammonium chloride (Merquat^{®} 550/Chemviron), polyaminopolyamides, and crosslinked water-soluble polymers thereof, cationic chitin derivatives, such as, for example, quaternized chitosan, optionally in microcrystalline dispersion, condensation products from dihaloalkyls, such as, for example, dibromobutane with bisdialkylamines, such as, for example, bis-dimethylamino-1,3-propane, cationic guar gum, such as, for example, Jaguar^{®} CBS, Jaguar^{®} C-17, Jaguar^{®} C-16 from Celanese, quaternized ammonium salt polymers, such as, for example, Mirapol^{®} A-15, Mirapol^{®} AD-1, Mirapol^{®} AZ-1 from Miranol.

Suitable anionic, zwitterionic, amphoteric and nonionic polymers are, for example, vinyl acetate-crotonic acid copolymers, vinylpyrrolidone-vinyl acrylate copolymers, vinyl acetate-butyl maleate-isobornyl acrylate copolymers, methyl vinyl ether-maleic anhydride copolymers and esters thereof, uncrosslinked polyacrylic acids and polyacrylic acids crosslinked with polyols, acrylamidopropyltrimethylammonium chloride-acrylate copolymers, octylacrylamide-methyl methacrylate-tert-butylaminoethyl methacrylate-2-hydroxypropyl methacrylate copolymers, polyvinylpyrrolidone, vinylpyrrolidone-vinyl acetate copolymers, vinylpyrrolidone-dimethylaminoethyl methacrylate-vinylcaprolactam terpolymers, and optionally derivatized cellulose ethers and silicones.

In one embodiment of the invention the cosmetic composition further comprises at least one silicone compound.

Suitable silicone compounds are, for example, dimethylpolysiloxanes, methylphenylpolysiloxanes, cyclic silicones, and amino-, fatty acid-, alcohol-, polyether-, epoxy-, fluorine-, glycoside- and/or alkyl-modified silicone compounds, which can either be liquid or in resin form at room temperature. Also suitable are simethicones, which are mixtures of dimethicones having an average chain length of from 200 to 300 dimethylsiloxane units and hydrogenated silicates.

In one embodiment of the invention the cosmetic composition further comprises at least one UV photoprotective filter.

UV photoprotective factors are, for example, to be understood as meaning organic substances (photoprotective filters) which are liquid or crystalline at room temperature and which are able to absorb ultraviolet rays and give off the absorbed energy again in the form of longer-wavelength radiation, e.g. heat. UVB filters can be oil-soluble or water-soluble. Examples of oil-soluble substances are:
- 3-benzylidenecamphor or 3-benzylidenenorcamphor and derivatives thereof, e.g. 3-(4-methylbenzylidene)camphor;
- 4-aminobenzoic acid derivatives, preferably 2-ethylhexyl 4-(dimethylamino)benzoate, 2-octyl 4-(dimethylamino)benzoate and amyl 4-(dimethylamino)benzoate;
- esters of cinnamic acid, preferably 2-ethylhexyl 4-methoxycinnamate, propyl 4-methoxycinnamate, isoamyl 4-methoxycinnamate, 2-ethylhexyl 2-cyano-3,3-phenylcinnamate (octocrylene);
- esters of salicylic acid, preferably 2-ethylhexyl salicylate, 4-isopropylbenzyl salicylate, homomenthyl salicylate;
- derivatives of benzophenone, preferably 2-hydroxy-4-methoxybenzophenone, 2-hydroxy-4-methoxy-4'-methylbenzophenone, 2,2'-dihydroxy-4-methoxybenzophenone;
- esters of benzalmalonic acid, preferably di-2-ethylhexyl 4-methoxybenzalmalonate;
- triazine derivatives, such as, for example, 2,4,6-trianilino(p-carbo-2'-ethyl-1'-hexyloxy)-1,3,5-triazine and octyltriazone or dioctylbutamidotriazone (Uvasorb^{®} HEB);
- propane-1,3-diones, such as, for example, 1-(4-tert-butylphenyl)-3-(4'-methoxyphenyl)propane-1,3-dione;
- ketotricyclo(5.2.1.0)decane derivatives.

Suitable water-soluble substances are:
- 2-phenylbenzimidazole-5-sulphonic acid and the alkali metal, alkaline earth metal, ammonium, alkylammonium, alkanolammonium and glucammonium salts thereof;
- sulphonic acid derivatives of benzophenones, preferably 2-hydroxy-4-methoxybenzophenone-5-sulphonic acid and its salts;
- sulphonic acid derivatives of 3-benzylidenecamphor, such as, for example, 4-(2-oxo-3-bomylidenemethyl)benzenesulphonic acid and 2-methyl-5-(2-oxo-3-bornylidene)sulphonic acid and salts thereof.

Suitable typical UV-A filters are, in particular, derivatives of benzoylmethane, such as, for example, 1-(4'-tert-butylphenyl)-3-(4'-methoxyphenyl)propane-1,3-dione, 4-tert-butyl-4'-methoxydibenzoylinethane (Parsol^{®} 1789), 1-phenyl-3-(4'-isopropylphenyl)propane-1,3-dione, and enamine compounds. The UV-A and UV-B filters can of course also be used in mixtures. Particularly favourable combinations consist of the derivatives of benzoylmethane, e.g. 4-tert-butyl-4'-methoxydibenzoylinethane (Parsol^{®} 1789) and 2-ethylhexyl 2-cyano-3,3-phenylcinnamate (octocrylene) in combination with esters of cinnamic acid, preferably 2-ethylhexyl 4-methoxycinnamate and/or propyl 4-methoxycinnamate and/or isoamyl 4-methoxycinnamate. Advantageously, such combinations are combined with water-soluble filters such as, for example, 2-phenylbenzimidazole-5-sulphonic acid and their alkali metal, alkaline earth metal, ammonium, alkylammonium, alkanolammonium and glucammonium salts.

As well as said soluble substances, insoluble light protection pigments, namely finely dispersed metal oxides or salts, are also suitable for this purpose. Examples of suitable metal oxides are, in particular, zinc oxide and titanium dioxide and also oxides of iron, zirconium, silicon, manganese, aluminium and cerium, and mixtures thereof. Salts which may be used are silicates (talc), barium sulphate or zinc stearate. The oxides and salts are used in the form of the pigments for skincare and skin-protective emulsions and decorative cosmetics. The particles here should have an average diameter of less than 100 nm, preferably between 5 and 50 nm and in particular between 15 and 30 nm. They can have a spherical shape, but it is also possible to use particles which have an ellipsoidal shape or a shape deviating in some other way from the spherical form. The pigments can also be surface-treated, i.e. hydrophilicized or hydrophobicized. Typical examples are coated titanium dioxides, such as, for example, titanium dioxide T 805 (Degussa) or Eusolex^{®} T2000 (Merck). Suitable hydrophobic coating agents are here primarily silicones and, specifically in this case, trialkoxyoctylsilanes or simethicones. In sunscreens, preference is given to using so-called micro- or nanopigments. Preference is given to using micronized zinc oxide.

In one embodiment of the invention the cosmetic composition further comprises at least one biogenic active ingredient and/or antioxidant.

Biogenic active ingredients are understood as meaning, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, such as, for example, prunus extract, bambara nut extract and vitamin complexes.

Antioxidants interrupt the photochemical reaction chain which is triggered when UV radiation penetrates the skin. Typical examples thereof are amino acids (e.g. glycine, histidine, tyrosine, tryptophan) and derivatives thereof, imidazoles (e.g. urocanic acid) and derivatives thereof, peptides, such as D,L-camosine, D-carnosine, L-carnosine and derivatives thereof (e.g. anserine), carotenoids, carotenes (e.g. α-carotene, β-carotene, lycopene) and derivatives thereof, chlorogenic acid and derivatives thereof, lipoic acid and derivatives thereof (e.g. dihydrolipoic acid), auro-thioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulphoximine compounds (e.g. buthionine sulphoximines, homocysteine sulphoximine, buthionine sulphones, penta-, hexa-, heptathionine sulphoximine) in very low tolerated doses (e.g. pmol to µmol/kg), and also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linoleic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (vitamin A palmitate), and coniferyl benzoate of gum benzoin, rutic acid and derivatives thereof, α-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, superoxide dismutase, zinc and derivatives thereof (e.g. ZnO, ZnSO₄) selenium and derivatives thereof (e.g. selenomethionine), stilbenes and derivatives thereof (e.g. stilbene oxide, trans-stilbene oxide) and the derivatives (salts, esters, ethers, sugars, nucleotides, nucleosides, peptides and lipids) of said active ingredients which are suitable according to the invention.

In one embodiment of the invention the cosmetic composition further comprises at least one anti-microbial agent and/or preservative.

Suitable antimicrobial agents are, in principle, all substances effective against gram-positive bacteria, such as, for example, 4-hydroxybenzoic acid and its salts and esters, N-(4-chlorophenyl)-N'-(3,4-dichlorophenyl)urea, 2,4,4'-trichloro-2'-hydroxydiphenyl ether (triclosan), 4-chloro-3,5-dimethylphenol, 2,2'-methylenebis(6-bromo-4-chlorophenol), 3-methyl-4-(1-methylethyl)phenol, 2-benzyl-4-chlorophenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, N-octylsalicylamide or N-decylsalicylamide.
Suitable preservatives are, for example, phenoxy ethanol, formaldehyde solution, parabens, pentanediol or sorbic acid, and the silver complexes known under the name Surfacins^{®}, and also the other classes of substance listed in Annex 6, Part A and B of the Cosmetics Directive.

In one embodiment of the invention the cosmetic composition further comprises at least one film former.

Customary film formers are, for example, chitosan, microcrystalline chitosan, quaternized chitosan, polyvinylpyrrolidone, vinylpyrrolidone-vinyl acetate copolymers, polymers of the acrylic acid series, quaternary cellulose derivatives, collagen, hyaluronic acid and salts thereof, and similar compounds.

In one embodiment of the invention the cosmetic composition further comprises at least one swelling agent.

The swelling agents for aqueous phases may be montmorillonites, clay mineral substances, Pemulen, and alkyl-modified Carbopol grades (Goodrich). Other suitable polymers and swelling agents are given in the review by R. Lochhead in Cosm. Toil. 108, 95 (1993).
In one embodiment of the invention the cosmetic composition further comprises at least one hydrotrophic agent.

To improve the flow behaviour, it is also possible to use hydrotropic agents, such as, for example, ethanol, isopropyl alcohol, or polyols. Polyols which are suitable here preferably have 2 to 15 carbon atoms and at least two hydroxyl groups. The polyols can also contain further functional groups, in particular amino groups, or be modified with nitrogen. Typical examples are
- glycerol;
- alkylene glycols, such as, for example, ethylene glycol, diethylene glycol, propylene glycol, butylene glycol, hexylene glycol, and polyethylene glycols with an average molecular weight of from 100 to 1 000 daltons;
- technical-grade oligoglycerol mixtures with a degree of self-condensation of from 1.5 to 10, such as, for example, technical-grade diglycerol mixtures with a diglycerol content of from 40 to 50% by weight;
- methylol compounds, such as, in particular, trimethylolethane, trimethylolpropane, trimethylolbutane, pentaerythritol and dipentaerythritol;
- lower alkyl glucosides, in particular those having 1 to 8 carbon atoms in the alkyl radical, such as, for example, methyl and butyl glucoside;
- sugar alcohols having 5 to 12 carbon atoms, such as, for example, sorbitol or mannitol,
- sugars having 5 to 12 carbon atoms, such as, for example, glucose or sucrose;
- amino sugars, such as, for example, glucamine;
- dialcohol amines, such as diethanolamine or 2-amino-1,3-propanediol.

The total amount of further components can be 1 to 50% by weight, preferably 5 to 40% by weight, based on the compositions. The compositions can be prepared by customary cold or hot processes; preference is given to using the phase-inversion temperature method.
The present invention will now be further illustrated by way of examples.

### EXAMPLES

### Example 1

6-Fluoro-DL-Tryptophan [CAS 7730-20-3] is available from Sigma-Aldrich. 4-Methyl-DL-Tryptophan [CAS 1954-45-6] is available from Sigma-Aldrich. 6-Methyl-DL-Tryptophan [CAS 2280-85-5] is available from Sigma-Aldrich. 5-Methoxy-DL-Tryptophan [CAS 28052-84-8] is available from Sigma-Aldrich. 5-Hydroxy-L-Tryptophan [CAS 4350-09-8] is available from Sigma-Aldrich.

### Example 2: Melanogenesis inhibition assay

Melanocytes (B16 cell line) are inoculated in standard medium of cell culture with fetal calf serum (FCS). After an incubation of 3 days at 37°C and CO₂=5%, growth medium is exchanged for standard medium with a range of concentrations for each compound to be tested and a control without ingredient. After an incubation of 3 days, the level of melanin is measured by recording the optical density at 475 nm. After washing the cells by a balanced salt, and homogenization in a solution of 0.1 M NaOH, the number of viable cells is determined by the level of cellular proteins (Bradford's method).

The results are expressed in % against control (cell culture medium without compound) as a mean on 2 to 3 assays, each in triplicate.

**Table 1: Rate of cellular proteins & melanin in % / control (mean of assays in triplicate):**

| | Dose % (w/v) | Protein level | Melanin level |
|---|---|---|---|
| Control | - | 100 | 100 |
| 6-Fluoro-DL-Tryptophan | 0.001 | 106 | 44 |
| | 0.003 | 108 | 10 |
| 4-Methyl-DL-Tryptophan | 0.001 | 97 | 56 |
| | 0.003 | 100 | 33 |
| 6-Methyl-DL-Tryptophan | 0.003 | 102 | 81 |
| | 0.01 | 100 | 39 |
| 5-Methoxy-DL-Tryptophan | 0.001 | 96 | 90 |
| | 0.003 | 92 | 65 |

The results demonstrated that tested compound according to formula (I) has decreased in a dose dependent manner the rate of melanin synthesis in melanocytes, without any cytotoxic effect.

### Example 3: Stability assay

5-hydroxy-L-tryptophan, 6-fluoro-DL-tryptophan, 4-methyl-DL-tryptophan were dissolved at 1 or 10 mg/ml in a solution aqueous buffer at ph 5.5 and pH 7 (citric acid 0.1 M, Na₂HPO₄ 0.2 M):ethanol 1:1 vol:vol. The solutions are kept away from day light and stored at room temperature. After 30, 60 and 120 days, the coloration of the solutions are visually assessed.

**Table 2: Coloration after 30, 60 and 120 days of storage at room temperature at pH 5.5 and 7.**

| | pH 5.5 | | | pH 7.0 | | |
|---|---|---|---|---|---|---|
| | 30 days | 60 days | 120 days | 30 days | 60 days | 120 days |
| 5-hydroxy-L-tryptophan | ++ | ++ | ++ | ++ | ++ | ++ |
| 6-fluoro-DL-tryptophan | - | - | - | - | - | - |
| 4-methyl-DL-tryptophan | - | - | - | - | - | - |

| | | | | | | |
|---|---|---|---|---|---|---|
| - : no coloration + : moderate coloration ++ : strong coloration Up to 120 days of incubation in protic solution at pH 5.5 and 7, 6-fluoro-DL-tryptophan and 4-methyl-DL-tryptophan have demonstrated a high stability whereas 5-hydroxy-L-tryptophan leads to the formation of a visible dark-brown coloration at 30 days. | | | | | | |

## Claims

1. Use of the substance of formula (I) 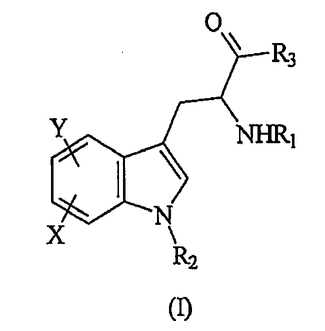 in the D-form, L-form or racemic form (D,L),
wherein
- R₁ is selected from the group consisting of hydrogen group (-H), methyl group (-CH₃), ethyl group (-CH₂CH₃), C3 alkyl group, C4 alkyl group, acetyl group (-C(O)CH₃), propanoyl group (-C(O)CH₂CH₃), n-butanoyl group (-C(O)CH₂CH₂CH₃), and 2-methyl-propanoyl group (-C(O)CH(CH₃)₂),
- R₂ is selected from the group consisting of hydrogen group (-H), ethyl group (-CH₂CH₃), C3 alkyl group, C4 alkyl group, acetyl group (-C(O)CH₃), propanoyl group (-C(O)CH₂CH₃), n-butanoyl group (-C(O)CH₂CH₂CH₃), and 2-methyl-propanoyl group (-C(O)CH(CH₃)₂),
- R₃ is selected from the group consisting of hydroxyl group (-OR), amino group (-NH₂), methoxy group (OCH₃), ethoxy group (-OCH₂CH₃), C3 alkoxy group, C4 alkoxy group, and benzyloxy group (-OCH₂C₆H₅),
- X and Y are independently selected from the group consisting of hydrogen group (-H), methyl group (-CH₃), ethyl group (-CH₂CH₃), C3 group alkyl, C4 alkyl group, halogen group, methoxy group (-OCH₃), ethoxy group (-OCH₂CH₃), C3 alkoxy group, C4 alkoxy group, and benzyloxy group (-OCH₂C₆H₅),
with the proviso that at least one of the residues X and Y is not a hydrogen group (-H),
(a) for the manufacture of cosmetic compositions and/or topical compositions
and/or
(b) in cosmetic compositions and/or topical compositions
for
(i) the lightening of the skin and/or
(ii) the whitening of the skin and/or
(iii) the reduction of pigmentation of the skin and/or
(iv) the reduction of hyperpigmentation of the skin and/or
(v) the inhibition of melanogenesis in the skin and/or
(vi) the prevention and/or retardation of signs of ageing and/or improving the skin appearance of an aged skin.

2. Use according to claim 1, wherein the substance according to formula (I) or a salt thereof is present in an amount of 0.0001 to 10 wt.-% based on the total amount of the composition.

3. Use according to claim 1 or 2, wherein
- R₁ is selected from the group consisting of hydrogen group (-H), methyl group (-CH₃), ethyl group (-CH₂CH₃), n-propyl group (-CH₂CH₂CH₃), and isopropyl group (-C(CH₃)₂),
- R₂ is selected from the group consisting of hydrogen group (-H), methyl group (-CH₃), ethyl group (-CH₂CH₃), n-propyl group (-CH₂CH₂CH₃), and isopropyl group (-C(CH₃)₂),
- R₃ is selected from the group consisting of hydroxyl group (-OH), amino group (-NH₂), methoxy group (OCH₃), ethoxy group (-OCH₂CH₃), and benzyloxy group (-OCH₂C₆H₅),
- X and Y are independently selected from the group consisting of hydrogen group (-H), methyl group (-CH₃), fluorine group (-F), bromine group (-Br), methoxy group (-OCH₃), and ethoxy group (-OCH₂CH₃),
with the proviso that at least one of the residues X and Y is not a hydrogen group (-H).

4. Use according to anyone of the preceding claims, wherein
- R₁ is either a hydrogen group (-H) or a methyl group (-CH₃),
- R₂ is either hydrogen group (-H) or methyl group (-CH₃),
- R₃ is either hydroxyl group (-OH) or amino group (-NH₂),
- Y is a hydrogen group (-H), and
- X is selected from the group consisting of methyl group (-CH₃), fluorine group (-F), and methoxy group (-OCH₃).

5. Use according to anyone of the preceding claims, wherein
- R₁ and R₂ are hydrogen groups (-H),
- R₃ is a hydroxyl group (-OH),
- Y is a hydrogen group (-H), and
- X is either a methyl group (-CH₃) or fluorine group (-F).

6. Substance according to formula (I) as defined in anyone of the preceding claims for the treatment of a disease connected to a disorder in the pigmentation of the skin.

7. Substance according to claim 6, wherein said disease exhibits an abnormal melanin synthesis and/or secretion.

8. Cosmetic and/or topical composition comprising a substance according to formula (I) or a salt thereof as defined in anyone of the preceding claims 1 to 5.
